# EUROPÄISCHE PATENTSCHRIFT

(11) **EP 0 883 590 B1**
(45) Veröffentlichungstag und Bekanntmachung des Hinweises auf die Patenterteilung: **02.05.2001**
(21) Anmeldenummer: 97905119.0
(22) Anmeldetag: 28.02.1997
(51) Int. Cl.: C07C 29/149, C07C 31/20

(54) **VERFAHREN ZUR HERSTELLUNG VON 1,6-HEXANDIOL MIT EINER REINHEIT ÜBER 99 %**
PROCESS FOR PREPARING 1,6 HEXANE DIOL WITH A LEVEL OF PURITY OVER 99 %
PROCEDE DE PREPARATION DE 1,6 HEXANEDIOL AVEC UN DEGRE DE PURETE SUPERIEUR A 99 %

(30) Priorität: 01.03.1996 DE 19607955; 15.11.1996 DE 19647348
(43) Veröffentlichungstag der Anmeldung: 16.12.1998
(73) Patentinhaber: BASF AKTIENGESELLSCHAFT, 67056 Ludwigshafen (DE)
(72) Erfinder: BAUR, Karl, Gerhard, D-67063 Ludwigshafen (DE); FISCHER, Rolf, D-69121 Heidelberg (DE); PINKOS, Rolf, D-67098 Bad Dürkheim (DE); STEIN, Frank, D-67098 Bad Dürkheim (DE); RUST, Harald, D-67435 Neustadt (DE); BREITSCHEIDEL, Boris, D-67117 Limburgerhof (DE)
(86) Internationale Anmeldenummer: EP9700980
(87) Internationale Veröffentlichungsnummer: WO9731882

(56) Entgegenhaltungen:
- EP-A- 0 661 255
- EP-A- 0 673 909
- DE-A- 2 819 593

## Beschreibung

Die Erfindung betrifft ein Verfahren zur Herstellung von 1,6-Hexandiol von mindestens 99 %iger Reinheit, das insbesondere im wesentlichen von 1,4-Cyclohexandiolen frei ist, aus einem Carbonsäuregemisch, das bei der Oxidation von Cyclohexan zu Cyclohexanon/Cyclohexanol mit Sauerstoff oder Sauerstoff enthaltenden Gasen und durch Wasserextraktion des Reaktionsgemisches erhalten wird durch Veresterung der Säuren, Auftrennung des Veresterungsgemisches in eine von l,4-Cyclohexandiolen freie Esterfraktion und eine die 1,4-Cyclohexandiole enthaltende Fraktion, Hydrierung der Esterfraktion und Reinigung des 1,6-Hexandiols durch Destillation.

1,6-Hexandiol stellt einen gesuchten Monomerbaustein dar, der überwiegend auf dem Polyester- und Polyurethansektor eingesetzt wird.

Die wäßrigen Lösungen von Carbonsäuren, die bei der Oxidation von Cyclohexan zu Cyclohexanol und Cyclohexanon (vgl. Ullmann's Encyclopedia of Industrial Chemistry, 5. Ed, 1987, Vol. A8, S. 2/9) als Nebenprodukte entstehen, im folgenden Dicarbonsäurelösung (DCL) genannt, enthalten (berechnet wasserfrei in Gew.-%) im allgemeinen zwischen 10 und 40 % Adipinsäure, zwischen 10 und 40 % 6-Hydroxycapronsäure, zwischen 1 und 10 % Glutarsäure, zwischen 1 und 10 % 5-Hydroxyvaleriansäure, zwischen 1 und 5 % 1,2-Cyclohexandiole (cis und trans), zwischen 1 und 5 % 1,4-Cyclohexandiol (cis und trans), zwischen 2 und 10 % Ameisensäure sowie eine Vielzahl weiterer Mono- und Dicarbonsäuren, Ester, Oxo- und Oxa-Verbindungen, deren Einzelgehalte im allgemeinen 5 % nicht übersteigen. Beispielhaft seien Essigsäure, Propionsäure, Buttersäure, Valeriansäure, Capronsäure, Oxalsäure, Malonsäure, Bernsteinsäure, 4-Hydroxybuttersäure und gamma-Butyrolacton genannt.

Aus DE 2 321 101 und DE-PSE 1 235 879 ist bekannt, diese wäßrigen Dicarbonsäurelösungen bei Temperaturen von 120 bis 300°C und Drucken von 50 bis 700 bar in Gegenwart überwiegend Kobalt enthaltender Katalysatoren zu 1,6-Hexandiol als Hauptprodukt zu hydrieren. Aus der EP-A-673 909 ist ein Verfahren zur Herstellung von 1,6-Hexandiol bekannt, bei dem die wäßrigen Dicarbonsäurelösungen zunächst verestert werden und die sich direkt anschließende Hydrierung des Veresterungsaustrags in Gegenwart einer Kombination aus zwei chromfreien Katalysatoren durchgeführt wird. Die Hydrierausträge werden in den vorgenannten Verfahren bevorzugt destillativ aufgearbeitet. Dabei gelingt es auch mit extrem hohem Destillationsaufwand nicht oder nur unvollständig, die bei der Hydrierung nicht veränderten 1,4-Cyclohexandiole von 1,6-Hexandiol zu trennen, so daß sich die 1,4-Cyclohexandiole, die anfänglich bereits in der DCL enthalten waren, mit einem Gehalt von im allgemeinen 2 bis 5 Gew.-% im 1,6-Hexandiol wiederfinden.

Um diesem Problem zu begegnen sind einige Lösungsansätze bekannt:

In US 3 933 930 wird die Umsetzung von 1,4-Cyclohexandiol in wäßrigen Lösungen von Adipinsäure und 6-Hydroxycapronsäure zu Cyclohexanol, Cyclohexan und/oder Cyclohexen beschrieben, indem das Gemisch katalytisch vorhydriert wird. Dieses Verfahren bedarf des Einsatzes zweier verschiedener Hydrierkatalysatoren, eines für die Vorhydrierung, eines für die eigentliche Carbonsäurehydrierung und ist daher aufwendig.

Nach DE-OS 2 060 548 wird sehr reines 1,6-Hexandiol durch Kristallisation gewonnen. Auch dieses Verfahren ist sehr aufwendig und außerdem mit erheblichen Ausbeuteverlusten verbunden.

Eine weitere Möglichkeit zur Gewinnung von hochreinem 1,6-Hexandiol besteht darin, anstelle von DCL reine Adipinsäure oder reine Adipinsäureester zu hydrieren (K. Weissermel, H.J. Arpe, Industrielle Organische Chemie, VCH-Verlagsgemeinschaft Weinheim, 4. Auflage, Seite 263, 1994). Reine Adipinsäure ist jedoch im Vergleich zu DCL sehr teuer. Ferner ist das Carbonsäuregemisch, das bei der Cyclohexanoxidation anfällt, ein Abfallprodukt, das auch aus Umweltschutzgesichtspunkten einer stofflichen Verwendung zugeführt werden sollte.

Es bestand daher die Aufgabe, ein neues Verfahren zu entwickeln, bei dem man 1,6-Hexandiol mit hoher Reinheit, in hoher Ausbeute und vertretbarem Aufwand aus DCL gewinnen kann.

Diese Aufgabe wurde erfindungsgemäß gelöst mit einem Verfahren zur Herstellung von Hexandiol-1,6 aus einem Adipinsäure, 6-Hydroxycapronsäure und in geringen Mengen 1,4-Cyclohexandiole enthaltenden Carbonsäuregemisch, das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemischs erhalten wird, durch Veresterung der Säuren und Hydrierung, wobei man
a) die in dem wäßrigen Dicarbonsäuregemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag in an sich bekannter Weise Hexandiol-1,6 gewinnt.

Es war überraschend, daß bei der Trennung der durch die Veresterung der in der DCL enthaltenen Mono- und Dicarbonsäuren entstandenen Estergemische, die 1,4-Cyclohexandiole, die ja ebenfalls mit Carbonsäuren verestert vorliegen können, so abgetrennt werden können, daß nach Hydrierung und Aufarbeitung dem verbleibenden sehr geringen 1,4-Cyclohexandiolgehalt im 1,6-Hexandiol keine praktische Bedeutung mehr zukommt. Wegen der zu trennenden komplizierten Stoffgemische war es überraschend, daß es gelang, die 1,4-Cyclohexandiole oder deren Ester, z.B. trotz der ungünstigen Siedepunktverhältnisse und der zu befürchtenden Azeotropbildung, praktisch vollständig von den für die Hydrierung zu 1,6-Hexandiol verwendeten C₆-Estern abzutrennen.

Die Veresterung kann ohne Zusatz von Katalysatoren bevorzugt unter Einwirkung von Katalysatoren durchgeführt werden. Als niedermolekulare Alkohole kommen in der Regel solche mit 1 bis 10 C-Atomen, insbesondere Alkanole mit 1 bis 8 C-Atomen in Betracht. Auch Diole wie Butandiol oder Pentandiol kommen prinzipiell in Betracht.

Die technisch bevorzugten für die Veresterung zu verwendenden Alkohole sind n- oder i-Butanol und insbesondere Methanol.

Im Falle der Veresterung mit Methanol (Variante A) geht man so vor, daß man in der Destillationsstufe c) eine im wesentlichen von 1,4-Cyclohexandiolen freie Carbonsäuremethylesterfraktion am Kopf der Kolonne und eine die Hochsieder und die 1,4-Cyclohexandiole enthaltende Sumpffraktion gewinnt und die Carbonsäuremethylesterfraktion in der Hydrierstufe (d) katalytisch hydriert.

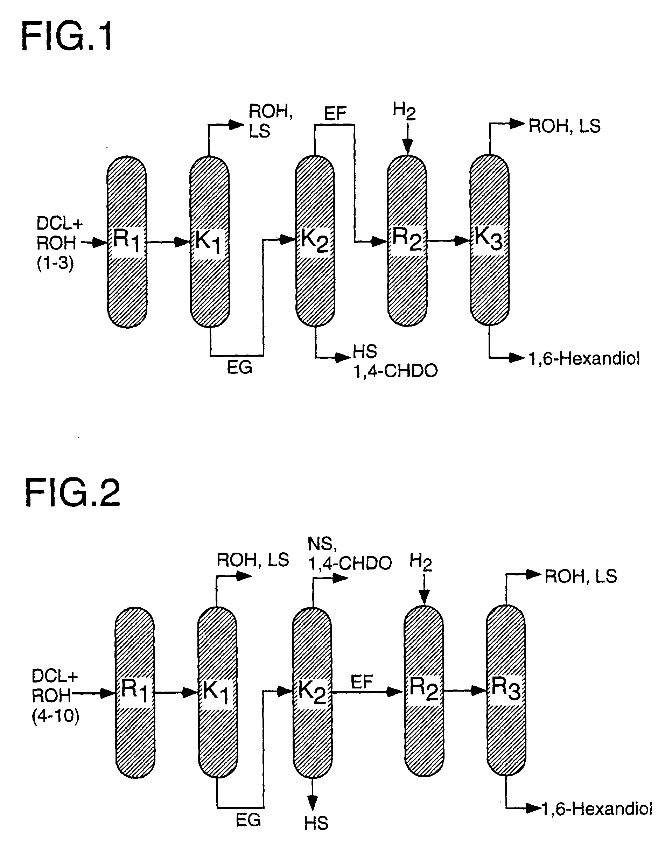

Wird n- oder i-Butanol zur Veresterung verwendet (Variante B), werden in der Destillationsstufe c) die 1,4-Cyclohexandiole mit den Leichtsiedern über Kopf abgetrennt und man gewinnt die Carbonsäurebutylester als Seitenabzug oder als diese enthaltenden Sumpf mit nachfolgender Einleitung in die Hydrierstufe (d).
Das erfindungsgemäße Verfahren wird mit seinen Varianten A (Fig. 1) und Variante B (Fig. 2) wie folgt allgemein erläutert (wobei die Begriffe über Kopf bzw. als Sumpf jeweils den Abzug oberhalb bzw. unterhalb des Zulaufs bedeuten):

### Variante A

Wie in Fig. 1 dargestellt, wird die Dicarbonsäurelösung (DCL), gegebenenfalls nach Entwässerung, zusammen mit einem C₁- bis C₃-Alkohol, vorzugsweise Methanol, in den Veresterungsreaktor R₁ eingespeist, wo die Carbonsäuren verestert werden. Das erhaltene Veresterungsgemisch gelangt dann in Kolonne K₁, in der der überschüssige Alkohol (ROH), Wasser und Leichtsieder (LS) über Kopf abdestilliert und das Estergemisch (EG) als Sumpf abgezogen und in die Fraktionierkolonne K₂ eingespeist wird. In dieser Kolonne wird das Gemisch in einer im wesentlichen von 1,4-Cyclohexandiolen freie Esterfraktion (EF) und eine Sumpffraktion, bestehend aus Hochsiedern (HS) und 1,4-Cyclohexandiolen (1,4-CHDO), fraktioniert. Die Esterfraktion (EF) wird dann in dem Hydrierreaktor R₂ katalytisch hydriert und das Hydriergemisch in der Destillationskolonne K₃ in Alkohol (ROH), Leichtsieder (LS) und reines 1,6-Hexandiol aufgetrennt.

### Variante B

Verwendet man zur Veresterung Alkohole mit 4 und mehr Kohlenstoffatomen, insbesondere n- oder i-Butanol, unterscheidet sich das Verfahren gemäß Fig. 2 nur insofern, als in der Fraktionskolonne K₂ das Estergemisch (EG) in ein Kopfprodukt von Niedrigsiedern (NS), die die 1,4-Cyclohexandiole (1,4-CHDO) enthalten, und eine im wesentlichen von 1,4-Cyclohexandiol freie Esterfraktion (EF) aufgetrennt wird, die man als Seitenfraktion oder als die Esterfraktion enthaltenden Sumpf gewinnt und in die Hydrierstufe (R₂) einspeist.

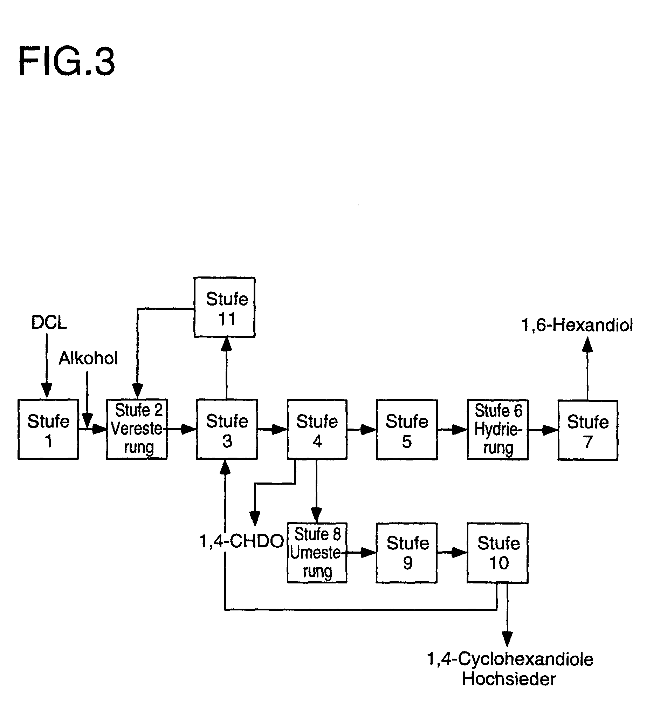

Das erfindungsgemäße Verfahren wird im einzelnen wie folgt näher erläutert. Dabei sind gemäß Fig. 3 die einzelnen Verfahrensschritte in weitere Stufen aufgeschlüsselt, wobei die Stufen 2, 3, 4, 5, 6, 7 für das Verfahren essentiell sind und die Stufen 3 und 4 sowie 6 und 7 auch zusammengefaßt werden können. Die Stufen 8, 9, 10 und 11 sind fakultativ, aber zur Erhöhung der Wirtschaftlichkeit des Verfahrens gegebenenfalls sinnvoll.

Die Dicarbonsäurelösung (DCL) ist im allgemeinen eine wäßrige Lösung mit einem Wasseranteil von 20 bis 80 %. Da eine Veresterungsreaktion eine Gleichgewichtsreaktion darstellt, ist es meist sinnvoll, insbesondere bei Veresterung mit z.B. Methanol, vorhandenes Wasser vor der Reaktion zu entfernen, vor allem, wenn während der Veresterungsreaktion Wasser nicht, z.B. nicht azeotrop, entfernt werden kann. Die Entwässerung in Stufe 1 kann z.B. mit einem Membransystem erfolgen, oder bevorzugt durch eine Destillationsapparatur, bei der bei 10 bis 250°C, bevorzugt 20 bis 200°C, besonders bevorzugt 30 bis 200°C und einem Druck von 1 bis 1500 mbar, bevorzugt 5 bis 1100 mbar, besonders bevorzugt 20 bis 1000 mbar Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, daß das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.% betragen.

Die Abtrennung des Wassers kann so erfolgen, daß das Wasser überwiegend säurefrei erhalten wird, oder man kann die in der DCL enthaltenen niederen Monocarbonsäuren - im wesentlichen Ameisensäure - zum größten Teil mit dem Wasser abdestillieren, damit diese in der Veresterung keinen Veresterungsalkohol binden.

Dem Carbonsäurestrom aus der Stufe 1 wird ein Alkohol mit 1 bis 10 C-Atomen zugemischt, gemäß Variante A Alkohole mit 1 bis 3 Kohlenstoffatomen, d.s. Methanol, Ethanol, Propanol oder iso-Propanol, bevorzugt Methanol, gemäß Variante B Alkohole mit 4 bis 10, insbesondere 4 bis 8 Kohlenstoffatomen und besonders bevorzugt n-Butanol, iso-Butanol, n-Pentanol und i-Pentanol.

Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor der Stufe 2, in dem die Carbonsäuren mit dem Alkohol verestert werden. Die Veresterungsreaktion kann bei 50 bis 400°C, bevorzugt bei 70 bis 300°C, besonders bevorzugt bei 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen; bevorzugt wird aber zur Erhöhung der Reaktionsgeschwindigkeit ein Katalysator zugesetzt. Dabei kann es sich um einen homogen gelösten oder um einen festen Katalysator handeln. Als homogene Katalysatoren seien beispielhaft Schwefelsäure, Phosphorsäure, Salzsäure, Sulfonsäuren, wie p-Toluolsulfonsäure, Heteropolysäuren wie Wolframatophosphorsäure oder Lewissäuren wie Aluminium-, Vanadium-, Titan-, Bor-Verbindungen genannt. Bevorzugt sind Mineralsäuren, insbesondere Schwefelsäure. Das Gew.-Verhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie SiO₂, Al₂O₃, SnO₂, ZrO₂ oder Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt, abzüglich der gegebenenfalls zugesetzten Säure als Katalysator, 0,01 bis 50, bevorzugt 0,1 bis 10. Dabei liegen nicht alle im System vorhandenen Carboxylgruppen als Ester des eingesetzten Alkohols vor, sondern ein Teil kann in Form von dimeren oder oligomeren Estern, z.B. mit dem OH-Ende der Hydroxycapronsäure vorliegen.

Das Veresterungsgemisch wird in Stufe 3, ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet.

Wird in Stufe 3 eine Kolonne verwendet, so erfolgt der Zulauf zur Kolonne bevorzugt zwischen dem Kopf- und dem Sumpfstrom. Über Kopf wird bei Drücken von 1 bis 1500 mbar, bevorzugt 20 bis 1000 mbar, besonders bevorzugt 40 bis 800 mbar und Temperaturen zwischen 0 und 150°C, bevorzugt 15 und 90°C, und insbesondere 25 und 75°C der überschüssige Veresterungsalkohol ROH, Wasser sowie z.B. entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren, wie Adipinsäure und Glutarsäure, Hydroxycarbonsäuren, wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 10 Gew.-% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 3 wird in die Stufe 4 eingespeist. Dabei handelt es sich um eine Destillationskolonne, bei der der Zulauf im allgemeinen zwischen den leichtsiedenden Komponenten und den schwersiedenden Komponenten erfolgt. Die Kolonne wird bei Temperaturen von 10 bis 300°C, bevorzugt 20 bis 270°C, besonders bevorzugt 30 bis 250°C und Drucken von 1 bis 1000 mbar, bevorzugt 5 bis 500 mbar, besonders bevorzugt 10 bis 200 mbar, betrieben.

Nach Variante A, d.h. der Veresterung mit C₁- bis C₃-Alkoholen, insbesondere Methanol, wird nun der Strom aus Stufe 3 in eine zu hydrierende Kopffraktion und eine die 1,4-Cyclohexandiole enthaltende Sumpffraktion getrennt.

Die Kopffraktion besteht überwiegend aus Restwasser und Restalkohol ROH, Estern des Alkohols ROH mit Monocarbonsäuren, überwiegend C₃- bis C₆-Monocarbonsäuren, Estern mit Hydroxycarbonsäuren, wie 6-Hydroxycapronsäure, 5-Hydroxyvaleriansäure, sowie vor allem den Diestern mit Dicarbonsäuren, wie Adipinsäure, Glutarsäure und Bernsteinsäure, ferner 1,2-Cyclohexandiolen, Caprolacton und Valerolacton.

Die genannten Komponenten können zusammen über Kopf abgetrennt und in die Hydrierung (Stufe 5) eingeschleust werden oder in einer weiteren bevorzugten Ausführungsform in der Kolonne in einen Kopfstrom, der überwiegend Restwasser und Restalkohol sowie die oben erwähnten Ester der C₃- bis C₅-Carbonsäuren enthält und einen Seitenstrom, der überwiegend die oben erwähnten Ester der C₆-Carbonsäuren und Dicarbonsäuren enthält, die dann in die Hydrierung gelangen, aufgetrennt werden.

Die schwersiedenden Komponenten des Stromes aus Stufe 4, überwiegend bestehend aus 1,4-Cyclohexandiolen oder deren Ester, dimeren oder oligomeren Estern sowie nicht näher definierten z.T. polymeren Bestandteilen der DCL, werden über den Abtriebsteil der Kolonne abgetrennt. Diese können zusammen anfallen oder so, daß über einen Seitenstrom der Kolonne im Abtriebsteil vorwiegend die 1,4-Cyclohexandiole und über Sumpf der Rest abgetrennt werden. Die so gewonnenen 1,4-Cyclohexandiole können z.B. als Ausgangsstoff für Wirkstoffe Verwendung finden. Die schwersiedenden Komponenten, mit oder ohne den Gehalt an 1,4-Cyclodiolen, können entweder verbrannt werden oder in einer bevorzugten Ausführungsform zur sog. Umesterung in die Stufe 8 gelangen.

Nach Variante B, d.h. der Veresterung mit C₄- bis C₁₀-Alkoholen, insbesondere n- oder i-Butanol, kann der Strom aus Stufe 3 in der Stufe 4 in eine die 1,4-Cyclohexandiole enthaltene Kopffraktion, einen vorwiegend die C₆-Ester enthaltenden Seitenstrom, der in die Hydrierung gelangt und Hochsieder enthaltenden Sumpfstrom, der gegebenenfalls in die Stufe 8 gelangen kann, aufgetrennt werden.

Die Kopffraktion besteht überwiegend aus Restalkohol ROH, C₁- bis C₃-Monoestern des Alkohols ROH, Valerolacton und 1,2- und 1,4-Cyclohexandiolen.

Der Seitenstrom enthält überwiegend Diester von Bernsteinsäure, Glutarsäure und Adipinsäure sowie Monoester der 5-Hydroxyvaleriansäure und 6-Hydroxycapronsäure. Dieser Seitenstrom kann entweder oberhalb oder auch unterhalb der Zulaufstelle der Kolonne entnommen werden und in die Hydrierung (Stufe 5) eingeschleust werden.

Der Sumpfstrom mit oligomeren Estern und sonstigen Hochsiedern kann analog der Variante A entweder verbrannt oder vorteilhaft in die Stufe 8 gelangen.

Nach einer weiteren Ausführungsform werden in der Stufe 4 die C₆-Ester zusammen mit entweder dem Sumpfstrom abgetrennt und dann, in einer weiteren Kolonne, entweder als Sumpfprodukt von der bereits beschriebenen Kopffraktion, die überwiegend aus Restalkohol ROH, C₁- bis C₃-Monoestern des Alkohols ROH, Valerolacton und 1,2-und 1,4-Cyclohexandiolen besteht, oder als Kopfstrom von den Hochsiedern abgetrennt.

Die von 1,4-Cyclohexandiole freie oder praktisch freie Fraktion der Stufe 4, entweder der Gesamtstrom oder der hauptsächlich Ester der C₆-Säuren enthaltende Seitenstrom, wird in die Hydrierstufe 5 geleitet.

Die Stufen 3 und 4 können, insbesondere wenn nur kleinere Mengen verarbeitet werden, zusammengefaßt werden. Dazu kann beispielsweise in einer absatzweise durchgeführten fraktionierten Destillation der C₆-Esterstrom gewonnen werden, wiederum ohne daß 1,4-Cyclohexandiole in den zur Hydrierung geführten Strom gelangen.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind z.B. in Houben-Weyl, Methoden der Organischen Chemie, Band IV/1c, Georg Thieme Verlag Stuttgart, 1980, S. 45 - 67) und Beispiele für heterogene Katalysatoren sind z.B. in Houben-Weyl, Methoden der Organischen Chemie, Band IV/1c, S. 16 bis 26 beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. Cr₂O₃, Al₂O₃, SiO₂, ZrO₂, ZnO₂, BaO und MgO oder Mischungen daraus.

Besonders bevorzugt sind Katalysatoren, wie sie in EP 0 552 463 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c ≧ 0, d > 0, a > b/2, b > a/4, a > c und a > d gilt und x die zur Wahrung der Elektronneutralität pro Formeleinheit erforderliche Anzahl von Sauerstoffionen bezeichnet. Die Herstellung dieser Katalysatoren kann beispielsweise nach den Angaben der EP 552 463 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so daß man als besonders bevorzugte Fällungsmittel Alkalicarbonate oder Ammoniumcarbonat einsetzt. Wichtig für die Herstellung der Katalysatoren ist die thermische Behandlung der Zwischenstufen bei Temperaturen zwischen 500°C und 1000°C. Die BET-Oberfläche der Katalysatoren liegt zwischen 10 und 150 m²/g.

Bevorzugt werden Heterogenkatalysatoren verwendet, die entweder fest angeordnet oder als Suspension eingesetzt werden. Wird die Hydrierung in der Gasphase und über fest angeordnetem Katalysator durchgeführt, werden im allgemeinen Temperaturen von 150 bis 300°C bei Drücken von 1 bis 100 bar, bevorzugt 15 bis 70 bar angewandt. Dabei wird zweckmäßig mindestens so viel Wasserstoff als Hydriermittel und Trägergas verwendet, daß Edukte, Zwischenprodukte und Produkte während der Reaktion nie flüssig werden. Der überschüssige Wasserstoff wird vorzugsweise im Kreis geführt, wobei ein kleiner Teil als Abgas zur Entfernung von Inerten wie z.B. Methan ausgeschleust werden kann. Es können dabei ein Reaktor oder mehrere Reaktoren hintereinander geschaltet verwendet werden.

Erfolgt die Hydrierung in der Flüssigphase mit fest angeordnetem oder suspendiertem Katalysator, so wird sie im allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinandergeschalteten Reaktoren durchgeführt werden. Die Hydrierung in Flüssigphase über ein Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden.

Die Hydrierung kann diskontinuierlich, bevorzugt kontinuierlich erfolgen.

Der Hydrieraustrag besteht im wesentlichen aus 1,6-Hexandiol und dem Alkohol ROH. Weitere Bestandteile sind, vor allem falls der gesamte leichtsiedende Strom der Stufe 4 gemäß Variante A eingesetzt wurde, 1,5-Pentandiol, 1,4-Butandiol, 1,2-Cyclohexandiole sowie kleine Mengen von Monoalkoholen mit 1 bis 6 C-Atomen und Wasser.

Dieser Hydrieraustrag wird in der Stufe 6, die z.B. ein Membransystem oder bevorzugt eine Destillationskolonne ist, in den Alkohol ROH, der zusätzlich den größten Teil der weiteren leichtsiedenden Komponenten enthält und einen Strom, der überwiegend 1,6-Hexandiol neben 1,5-Pentandiol und den 1,2-Cyclohexandiolen enthält, aufgetrennt. Dabei werden bei einem Druck von 10 bis 1500 mbar, bevorzugt 30 bis 1200 mbar, besonders bevorzugt 50 bis 1000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt. Der leichtsiedende Stoffstrom kann entweder direkt in die Veresterung der Stufe 2 zurückgeführt werden oder in die Stufe 8 oder in die Stufe 11 gelangen.

Der 1,6-Hexandiol enthaltene Stoffstrom wird in der Stufe 7 in einer Kolonne gereinigt. Dabei werden 1,5-Pentandiol, gegebenenfalls die 1,2-Cyclohexandiole, sowie weitere eventuell vorhandene Leichtsieder, über Kopf abgetrennt. Sollen die 1,2-Cyclohexandiole und/oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust. 1,6-Hexandiol wird mit einer Reinheit von mindestens 99 % aus einem Seitenstrom der Kolonne entnommen. Dabei werden bei Drücken von 1 bis 1000 mbar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Sollen nur kleinere Mengen 1,6-Hexandiol hergestellt werden, so können die Stufen 6 und 7 auch in einer diskontinuierlichen fraktionierten Destillation zusammengefaßt werden.

Um das erfindungsgemäße Verfahren möglichst wirtschaftlich zu betreiben, ist es sinnvoll, den Veresterungsalkohol ROH zurückzugewinnen und immer wieder zur Veresterung einzusetzen. Dazu kann der vorwiegend den Alkohol ROH, beispielsweise Methanol enthaltende Strom aus Stufe 3 und/oder 6 in der Stufe 11 aufgearbeitet werden. Zu diesem Zweck wird vorteilhaft eine Kolonne verwendet, in der Komponenten, die leichter sieden als der Alkohol ROH über Kopf, Wasser und Komponenten, die höher sieden als der Alkohol ROH, über Sumpf vom Alkohol ROH, der in einem Seitenstrom gewonnen wird, abgetrennt werden. Die Kolonne wird zweckmäßig bei 500 bis 5000 mbar, bevorzugt bei 800 bis 3000 mbar, betrieben.

Nach einer weiteren bevorzugten Ausführungsform des erfindungsgemäßen Verfahrens wird der hochsiedende Strom aus Stufe 4 (gemäß Variante A) zur Erhöhung der Gesamtausbeute an 1,6-Hexandiol bezogen auf eingesetzte Adipinsäure und 6-Hydroxycapronsäure in der eingesetzten DCL, verwendet. Dazu wird in der Stufe 8 der Anteil an dimeren und oligomeren Estern der Adipinsäure bzw. Hydroxycapronsäure mit weiteren Mengen des Alkohols ROH in Gegenwart eines Katalysators umgesetzt. Das Gew.-Verhältnis von Alkohol ROH und dem Sumpfstrom aus Stufe 4 beträgt zwischen 0,1 bis 20, bevorzugt 0,5 bis 10, besonders bevorzugt 1 bis 5. Als Katalysatoren eignen sich prinzipiell die bereits für die Veresterung in Stufe 2 beschriebenen. Bevorzugt werden jedoch Lewissäuren eingesetzt. Beispiele hierzu sind Verbindungen oder Komplexe des Aluminiums, Zinns, Antimons, Zirkons oder Titans, wie Zirkoniumacetylacetonat oder Tetraalkyltitanate, z.B. Tetraisopropyltitanat, die in Konzentrationen von 1 bis 10000 ppm, bevorzugt 50 bis 6000 ppm, besonders bevorzugt 100 bis 4000 ppm, bezogen auf das Umesterungsgemisch, angewandt werden. Besonders bevorzugt hierbei sind Titanverbindungen.

Die Umesterung kann absatzweise oder kontinuierlich, in einem Reaktor oder mehreren Reaktoren, in Reihe geschaltenen Rührkesseln oder Rohrreaktoren bei Temperaturen zwischen 100 und 300°C, bevorzugt 120 bis 270°C, besonders bevorzugt 140 bis 240°C und den sich dabei einstellenden Eigendrücken, durchgeführt werden. Die benötigten Verweilzeiten liegen bei 0,5 bis 10 Stunden, bevorzugt bei 1 bis 4 Stunden.

Dieser Strom aus der Stufe 8 läßt sich im Falle der Veresterung mit Methanol z.B. wieder in die Stufe 3 einschleusen. Zur Vermeidung von Aufpegelungen, vor allem von 1,4-Cyclohexandiolen, muß dann absatzweise oder kontinuierlich ein Teilstrom der Hochsieder aus Stufe 4 ausgeschleust werden. Eine andere Möglichkeit ist, den Strom aus Stufe 8 nicht in Stufe 3 zurückzuführen, sondern ihn, analog zur Stufe 3, in einer Stufe 9 in vorwiegend Alkohol ROH, der dann wieder in die Stufe 2, 8 oder 11 gelangen kann, und einen Strom, der die Ester enthält, aufzutrennen.

Dieser Esterstrom kann prinzipiell (mit der Maßgabe der Vermeidung von Aufpegelungen der 1,4-Cyclohexandiole) in die Stufe 4 zurückgeführt werden oder wird bevorzugt in eine weitere Stufe 10, in die Ester der C₆-Säuren und, mengenmäßig eher unbedeutend, in die Ester der C₅-Säuren einerseits, die entweder in die Stufe 4 oder direkt in die Stufe 5 eingeschleust werden können und Hochsieder andererseits, die die 1,4-Cyclohexandiole enthalten, aufgetrennt, worauf die Hochsieder ausgeschleust werden.

Auf diese Weise lassen sich Ausbeuten an 1,6-Hexandiol von über 95 %, bei Reinheiten von über 99 % erzielen.

Das neue Verfahren erlaubt somit in wirtschaftlicher Weise aus einem Abfallprodukt hochreines 1,6-Hexandiol mit hoher Ausbeute zu gewinnen.

Das Verfahren wird anhand des nachfolgenden Beispiels näher erläutert aber in keiner Weise eingeschränkt.

### Beispiel (Variante A)

### Stufe 1 (Entwässerung):

0,1 kg/h Dicarbonsäurelösung (bestehend im wesentlichen aus Adipinsäure, 6-Hydroxycapronsäure, 1,4-Cyclohexandiolen, Glutarsäure, 5-Hydroxyvaleriansäure, Ameisensäure, Wasser) wurden kontinuierlich in eine Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden,) mit aufgesetzter Füllkörperkolonne (ca. 4 theoretische Trennstufen, kein Rücklauf am Kopf) destilliert. Als Kopfprodukt wurden 0,045 kg/h erhalten mit einem Ameisensäuregehalt im Wasser von ca. 3 %. Im Sumpfstrom (5,5 kg) betrug der Wassergehalt ca. 0,4 %.

### Stufe 2 (Veresterung):

5,5 kg/h des Sumpfstroms aus Stufe 1 wurden mit 8,3 kg/h Methanol und 14 g/h Schwefelsäure kontinuierlich in einem Rohrreaktor (l 0,7 m, 0̸ 1,8 cm, Verweilzeit 2,7 h) umgesetzt. Die Säurezahl des Austrags abzüglich Schwefelsäure betrug ca. 10 mg KOH/g.

### Stufe 3 (Entfernen überschüssigen Alkohols und von Wasser):

In einer 20 cm Füllkörperkolonne wurde der Veresterungsstrom aus Stufe 2 destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 4 (Fraktionierung; 1,4-Cyclohexandiolabtrennung):

In einer 50 cm Füllkörperkolonne wurde der Sumpfstrom aus Stufe 3 fraktioniert destilliert (1 mbar, 70-90°C Kopftemperatur, bis 180°C Sumpftemperatur). Der Sumpf (1,9 kg) enthielt praktisch alle 1,4-Cyclohexandiole.

Als Leichtsieder wurden 0,6 kg abdestilliert (1,2-Cyclohexandiole, Valerolacton, 5-Hydroxyvaleriansäuremethylester, Glutarsäuredimethylester, Bernsteinsäuredimethylester u.a.). Als überwiegend Adipinsäuredimethylester und 6-Hydroxycapronsäuremethylester enthaltende Fraktion wurden 4,3 kg erhalten.

Der die Esterfraktion darstellende Kopfstrom wird in Hydrierstufe 5 geleitet.

### Stufe 5 (Hydrierung):

4,3 kg der C₆-Esterfraktion aus Stufe 4 wurden kontinuierlich in einem 25-ml-Reaktor an einem Katalysator hydriert (Katalysator, 70 Gew.-% CuO, 25 Gew.-% ZnO, 5 Gew.-% Al₂O₃), der zuvor im Wasserstoffstrom bei 180°C aktiviert worden war. Der Zulauf betrug 20 g/h, der Druck 220 bar und die Temperatur 220°C). Der Ester-Umsatz betrug 99,5 %, die 1,6-Hexandiolselektivität betrug über 99 %.

Alternativ wurde die Esterfraktion in einer zweistufigen Reaktorkaskade (1. Reaktor 2,5 l Katalysator, Rieselfahrweise, 250 bar, Produktrückführung : Zulauf = 10 : 1, 220 - 230°C; 2. Reaktor 0,5 l Katalysator, Rieselfahrweise gerader Durchgang, 260 bar, 220°C) kontinuierlich hydriert. Als Katalysator wurde ein zuvor bei 180°C aktivierter Katalysator aus CuO (60 %), Al₂O₃ (30 %) und Mn₂O₃ (10 %) eingesetzt. Die Zulaufmenge betrug 1 kg/h. Bei 99,5 % Umsatz betrug die Hexandiol-Selektivität über 99 %.

### Stufe 6 und 7:

4,0 kg des Hydrieraustrags aus Stufe 5 wurden fraktioniert destilliert (Destillationsblase mit aufgesetzter 70 cm Füllkörperkolonne, Rücklaufverhältnis 2) Bei 1013 mbar wurde 1 kg Methanol abdestilliert. Nach Anlegen von Vakuum (20 mbar) destillierten überwiegend die 1,2-Cyclohexandiole und 1,5-Pentandiol ab. Danach (Sdp. 146°C) destillierte 1,6-Hexandiol mit einer Reinheit von 99,8 % ab. (Restgehalt überwiegend 1,5-Pentandiol.)

### Stufe 8:

1,9 kg des Sumpfaustrages der Stufe 4 wurden mit 3,8 kg Methanol und 3,8 g Tetra-i-propyltitanat versetzt und kontinuierlich in einem 1 m langen, 440 ml fassenden Rohrreaktor, der mit 3 mm V2A-Ringen gefüllt war, umgesetzt. Die mittlere Verweilzeit betrug ca. 2 h.

### Stufe 9:

Der Austrag aus Stufe 8 wurde analog der in Stufe 3 beschriebenen Apparatur fraktioniert destilliert. Bei 65°C Kopftemperatur wurden 3,5 kg abdestilliert (überwiegend Methanol). Im Sumpf verblieben 2,2 kg.

### Stufe 10:

Der Sumpf aus Stufe 9 wurde analog Stufe 4 bis zu einer Sumpftemperatur von 160°C fraktioniert destilliert. Als Destillat wurden 1,3 kg erhalten, das direkt hydriert oder in die Stufe 4 zurückgeführt werden kann. Zusammensetzung: 52 % 6-Hydroxycapronsäuremethylester, 31 % Adipinsäuredimethylester, 5 % Glutarsäuredimethylester, 4 % 5-Hydroxycapronsäuremethylester sowie eine Vielzahl weiterer, mengenmäßig unbedeutender Komponenten.

### Stufe 11:

7 kg des Kopfproduktes der Stufe 3 wurden an einer 20 cm Füllkörperkolonne bei 1015 mbar fraktioniert destilliert. Es wurden 0,8 kg Vorlauffraktion bei 59-65°C Kopftemperatur erhalten, die neben vorwiegend Methanol, C₁-C₄-Monomethylester enthielt. Bei 65°C Kopftemperatur wurden 5,6 kg Methanol mit einer Reinheit > 99 % erhalten. Der Sumpf (0,6 kg) bestand überwiegend aus Wasser.

## Patentansprüche

1. Verfahren zur Herstellung von Hexandiol-1,6 aus einem Adipinsäure, 6-Hydroxycapronsäure und geringe Mengen 1,4-Cyclohexandiole enthaltenden Carbonsäuregemisch, das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemischs erhalten wird, durch Veresterung der Säuren und Hydrierung, dadurch gekennzeichnet, daß man
a) die in dem wäßrigen Dicarbonsäuregemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag in an sich bekannter Weise Hexandiol-1,6 gewinnt.

2. Verfahren gemäß Anspruch 1, dadurch gekennzeichnet, daß man das Carbonsäuregemisch vor der Veresterung entwässert.

3. Verfahren gemäß Anspruch 1, dadurch gekennzeichnet, daß man die Veresterung mit Alkanolen mit 1 bis 3 Kohlenstoffatomen durchführt.

4. Verfahren gemäß Anspruch 1, dadurch gekennzeichnet, daß man die Veresterung mit Alkanolen mit 4 bis 10 Kohlenstoffatomen durchführt.

5. Verfahren gemäß Anspruch 1, dadurch gekennzeichnet, daß man die Veresterung mit Methanol durchführt und in der Destillationsstufe (c) eine im wesentlichen von 1,4-Cyclohexandiolen freie Carbonsäuremethylesterfraktion am Kopf der Kolonne und eine die Hochsieder und die 1,4-Cyclohexandiole enthaltende Sumpffraktion gewinnt und die Carbonsäuremethylesterfraktion in der Hydrierstufe (d) katalytisch hydriert.

6. Verfahren gemäß Anspruch 1, dadurch gekennzeichnet, daß man die Veresterung mit n- oder i-Butanol durchführt und in der Destillationsstufe (c) die 1,4-Cyclohexandiole mit den Leichtsiedern über Kopf abtrennt und die Carbonsäurebutylester als Seitenabzug oder als diese enthaltenden Sumpf gewinnt und in der Hydrierstufe (d) katalytisch hydriert.

7. Verfahren gemäß Anspruch 1, dadurch gekennzeichnet, daß man aus dem den nicht umgesetzten Alkohol enthaltenden Kopfprodukt der Destillationsstufe (b) den Alkohol in reiner Form gewinnt und in die Veresterungsstufe (a) zurückführt.

8. Verfahren gemäß Anspruch 1, dadurch gekennzeichnet, daß man das Sumpfprodukt der Stufe (c) zumindest teilweise einer erneuten Veresterung unter weiterem Zusatz des niedermolekularen Alkohols und eines Veresterungskatalysators unterwirft und in einer getrennten Destillationsstufe analog (b) und (c) auftrennt, oder erst nach Abtrennung der 1,4-Cyclohexandiole die erneute Veresterung durchführt und die die Carbonsäureester enthaltende Fraktion in die Hydrierstufe (d) einleitet.

9. Verfahren gemäß Anspruch 1, dadurch gekennzeichnet, daß man zur Hydrierung Katalysatoren verwendet, die als katalytisch aktive Hauptbestandteile Kupfer, Kobalt und/oder Rhenium enthalten.

10. Verfahren nach Anspruch 1, dadurch gekennzeichnet, daß man zur Hydrierung Katalysatoren verwendet, die in der oxidischen Form die Zusammensetzung CuₐAl_{b}Zr_{c}Mn_{d}Oₓ besitzen, wobei a > 0, b > 0, c ≧ 0, d > 0, a > b/2, b > a/4, a > c und a > d gilt und x die zur Wahrung der Elektroneutralität pro Formeleinheit erforderliche Anzahl von Sauerstoffionen bezeichnet.

## Claims

1. A process for preparing 1,6-hexanediol from a carboxylic acid mixture which contains adipic acid, 6-hydroxycaproic acid and small amounts of 1,4-cyclohexanediols and which is obtained as byproduct of the oxidation of cyclohexane to cyclohexanone/cyclohexanol with oxygen or oxygen-containing gases and by extraction of the reaction mixture with water, by esterification of the acids and hydrogenation, wherein
a) the mono- and dicarboxylic acids present in the aqueous dicarboxylic acid mixture are reacted with a low molecular weight alcohol to give the corresponding carboxylic esters,
b) the resulting esterification mixture is freed of excess alcohol and low boilers in a first distillation stage,
c) the bottom product is fractionated in a second distillation stage into an ester fraction which is essentially free of 1,4-cyclohexanediols and a fraction containing at least the major part of the 1,4-cyclohexanediols,
d) the ester fraction which is essentially free of 1,4-cyclohexanediols is catalytically hydrogenated and
e) 1,6-hexanediol is isolated from the discharge from the hydrogenation in a conventional final distillation stage.

2. A process as claimed in claim 1, wherein the carboxylic acid mixture is dehydrated before the esterification.

3. A process as claimed in claim 1, wherein the esterification is carried out with alkanols having 1 to 3 carbon atoms.

4. A process as claimed in claim 1, wherein the esterification is carried out with alkanols having 4 to 10 carbon atoms.

5. A process as claimed in claim 1, wherein the esterification is carried out with methanol and in distillation stage (c) a methyl carboxylate fraction which is essentially free of 1,4-cyclohexanediols is obtained as distillate, the bottom fraction contains the high boilers and the 1,4-cyclohexanediols, and the methyl carboxylate fraction is catalytically hydrogenated in hydrogenation stage (d).

6. A process as claimed in claim 1, wherein the esterification is carried out with n- or i-butanol, and in distillation stage (c) the 1,4-cyclohexanediols are removed as distillate with the low-boilers, and the butyl carboxylates are obtained as side cut or in the bottom product and are catalytically hydrogenated in hydrogenation stage (d).

7. A process as claimed in claim 1, wherein alcohol is isolated in pure form from the distillate containing the unreacted alcohol from distillation stage (b) and is returned to the esterification stage (a).

8. A process as claimed in claim 1, wherein the bottom product from stage (c) is at least partly subjected to renewed esterification with further addition of the low molecular weight alcohol and an esterification catalyst, and is fractionated in a separate distillation stage similar to (b) and (c), or the renewed esterification is carried out only after removal of the 1,4-cyclohexanediols, and the fraction containing the carboxylic esters is passed to the hydrogenation stage (d).

9. A process as claimed in claim 1, wherein the catalysts used for the hydrogenation contain as catalytically active main ingredients copper, cobalt and/or rhenium.

10. A process as claimed in claim 1, wherein the catalysts used for the hydrogenation have, in the oxide form, the composition CuₐAl_{b}Zr_{c}Mn_{d}Oₓ, where a > 0, b > 0, c ≧ 0, d > 0, a > b/2, b > a/4, a > c and a > d, and x designates the number of oxygen ions necessary to ensure electroneutrality for each formula unit.

## Revendications

1. Procédé de préparation de l'hexanediol-1,6 à partir d'un mélange d'acides carboxyliques contenant de l'acide adipique, de l'acide 6-hydroxycaproïque et de faibles quantités de 1,4-cyclohexane-diols, que l'on obtient comme produit secondaire lors de l'oxydation du cyclohexane en cyclohexanone/cyclohexanol avec de l'oxygène ou des gaz contenant de l'oxygène et par extraction avec de l'eau du mélange réactionnel, par estérification des acides et hydrogénation, caractérisé en ce que,
a) l'on convertit les acides mono- et di-carboxyliques contenus dans le mélange aqueux d'acides dicarboxyliques avec un alcool de faible masse moléculaire en les esters carboxyliques correspondants,
b) dans une première étape de distillation, on élimine, du mélange d'estérification, l'alcool en excès et des substances à faible point d'ébullition,
c) dans une deuxième étape de distillation, en partant du produit de fond, on réalise une séparation en une fraction ester essentiellement exempte de 1,4-cyclohexanediols et en une fraction contenant au moins la plus grande partie des 1,4-cyclohexanediols,
d) on fait subir une hydrogénation catalytique à la fraction ester essentiellement exempte de 1,4-cyclohexanediols et
e) dans une étape de distillation pure, on extrait l'hexanediol-1,6 à partir du produit d'hydrogénation d'une façon connue en elle-même.

2. Procédé selon la revendication 1, caractérisé en ce que l'on déshydrate le mélange d'acides carboxyliques avant l'estérification.

3. Procédé selon la revendication 1, caractérisé en ce que l'on réalise l'estérification avec des alcanols ayant 1 à 3 atomes de carbone.

4. Procédé selon la revendication 1, caractérisé en ce que l'on réalise l'estérification avec des alcanols ayant 4 à 10 atomes de carbone.

5. Procédé selon la revendication 1, caractérisé en ce que l'on réalise l'estérification avec du méthanol et que dans l'étape de distillation (c), on extrait une fraction d'ester méthylique d'acide carboxylique essentiellement exempte de 1,4-cyclohexanediols en tête de colonne et une fraction de fond contenant les substances à point d'ébullition élevé et les 1,4-cyclohexanediols et on fait subir une hydrogénation catalytique à la fraction d'ester méthylique d'acide carboxylique dans l'étape d'hydrogénation (d).

6. Procédé selon la revendication 1, caractérisé en ce que l'on réalise l'estérification avec le n- ou i-butanol et, dans l'étape de distillation (c), on sépare les 1,4-cyclohexanediols d'avec les substances à faible point d'ébullition de tête et que l'on extrait l'ester butylique d'acide carboxylique en tant que produit de soutirage latéral ou en tant que produit de fond contenant celui-ci et on lui fait subir une hydrogénation catalyyique dans l'étape d'hydrogénation (d).

7. Procédé selon la revendication 1, caractérisé en ce que l'on extrait l'alcool sous forme pure à partir du produit de tête dans l'étape de distillation (b), contenant l'alcool n'ayant pas réagi et on le recycle dans l'étape d'estérification (a).

8. Procédé selon la revendication 1, caractérisé en ce que l'on soumet, au moins partiellement, le produit de fond de l'étape (c) à une nouvelle estérification avec un ajout supplémentaire de l'alcool de faible masse moléculaire et d'un catalyseur d'estérification et que l'on sépare dans une étape de distillation séparée analogue à (b) et (c), ou bien on réalise la nouvelle estérification qu'après la séparation des 1,4-cyclohexanediols et on introduit la fraction contenant les esters carboxyliques dans l'étape d'hydrogénation (d).

9. Procédé selon la revendication 1, caractérisé en ce que l'on utilise pour l'hydrogénation des catalyseurs qui contiennent en tant que composants principaux à action catalytique, le cuivre, le cobalt et/ou le rhénium.

10. Procédé selon la revendication 1, caractérisé en ce que l'on utilise pour l'hydrogénation des catalyseurs, qui possèdent, dans la forme oxydée, la composition CuₐAl_{b}Zr_{c}Mn_{d}Oₓ, où a > 0, b > 0, c ≥ 0, d > 0, a > b/2, b > a/4, a > c et a > d, et x représente le nombre d'ions oxygène nécessaire à la conservation de la neutralité électrique par unité de formule.
